# EUROPEAN PATENT APPLICATION

(11) **EP 1 561 455 A1**
(43) Date of publication of application: **10.08.2005**
(21) Application number: 03811134.0
(22) Date of filing: 13.11.2003
(51) Int. Cl.: A61K 7/32, A61K 7/00, A61K 7/48

(54) **COSMETIC COMPOSITION**

(30) Priority: 14.11.2002 JP 2002330609
(71) Applicant: Kao Corporation, Tokyo 103-8210 (JP)
(72) Inventor: KITO, Tetsuji, c/o Kao Corporation, Wakayama-shi, Wakayama 640-8580 (JP); NAMBU, Hiromi, c/o Kao Corporation, Wakayama-shi, Wakayama 640-8580 (JP); HAMAGUCHI, Takeshi, c/o Kao Corporation, Wakayama-shi, Wakayama 640-8580 (JP)
(74) Representative: HOFFMANN EITLE
(86) International application number: PCT/JP2003/014448
(87) International publication number: WO 2004/043420

(57) **Abstract**

The invention provides an emulsified cosmetic composition having an anti-perspiring action. The invention relates to an emulsified cosmetic composition comprising surface-hydrophobated water-absorbing polymer particles and water, particularly an emulsified cosmetic composition as an antiperspirant.

## Description

### Field of the invention

The present invention relates to an emulsified cosmetic composition useful as a skin agent for external application, particularly an emulsified cosmetic composition for antiperspiration.

### Background of the invention

In perspiring sites, particularly the armpit, stickiness due to perspiration worsens skin feel and an unpleasant smell is generated, and thus cosmetics having an anti-perspiring effect have been used. Particularly, an aluminum compound is used as a main component exhibiting an anti-perspiring effect (JP-A 52-99236). However, such a compound has gritty feel and is thus not satisfactory in respect of feel.

On one hand, emulsified cosmetics for face or body blended with a moisture-retaining component such as glycerin are used for the purpose of preventing drying of the skin. In the case of perspiring after application of such cosmetics, however, the emulsified cosmetics have no ability for antiperspiration, thus causing stickier feel attributable to the moisture-retaining component such as glycerin than in a non-coated state.

JP-A 5-70322 discloses water-in-oil emulsified cosmetics containing a water-absorbing polymer and dimethyl polysiloxane. However, the surface of the water-absorbing polymer used therein is not hydrophobated.

Cosmetics containing a water-absorbing organic polymer (JP-A 50-126820) are known to reduce stickiness due to perspiration, but the surface of the organic polymer is hydrophilic so that its effect on reduction of stickiness is not sufficient, and thus sticky feel is often caused.

### Summary of the invention

The present invention relates to an emulsified cosmetic composition having an anti-perspiring action.

The present invention provides an emulsified cosmetic composition containing surface-hydrophobated water-absorbing polymer particles and water, particularly an emulsified cosmetic composition as an antiperspirant.

The present invention provides use of an emulsified composition containing surface-hydrophobated water-absorbing polymer particles and water as cosmetics. The present invention also provides a method of antiperspiration, which includes applying an emulsified composition containing surface-hydrophobated water-absorbing polymer particles and water to the skin.

### Detailed description of the invention

### [Surface-hydrophobated water-absorbing polymer particles]

The surface-hydrophobated water-absorbing polymer particles used in the present invention may be water-absorbing polymer particles wherein at least a part of the surfaces of water-absorbing polymer particles was hydrophobated. Such surface-hydrophobated water-absorbing polymer particles include, for example, (1) surface-hydrophobated water-absorbing polymer particles wherein the surfaces of water-absorbing polymer particles were coated with a silicone compound having at least one kind of functional group, (2) surface-hydrophobated water-absorbing polymer particles wherein the surfaces of water-absorbing polymer particles were coated with a polymer of a hydrophobic vinyl monomer, (3) surface-hydrophobated water-absorbing polymer particles wherein the surfaces of water-absorbing polymer particles were treated with a fluorine-based surfactant, and (4) surface-hydrophobated water-absorbing polymer particles wherein the surfaces of water-absorbing polymer particles were treated with a silane coupling agent. Among these polymer particles, the surface-hydrophobated water-absorbing polymer particles
wherein the surfaces of water-absorbing polymer particles were coated with a silicone compound having at least one kind of functional group are preferable.

The shape of the surface-hydrophobated water-absorbing polymer particles of the present invention is not particularly limited, and may be not only in the form of sphere, egg or lump, etc., but also in the form of scale, plate, fiber, fine-powder agglomerate (granule) or in an amorphous state. Preferably, the surface-hydrophobated water-absorbing polymer particles are in the form of sphere, egg or lump for easy availability, more preferably in the form of sphere for good feeling. These particles may be porous.

When the surface-hydrophobated water-absorbing polymer particles of this invention are in the form of sphere, egg or lump, the average particle diameter thereof is preferably 0.1 µm or more, more preferably 0.5 µm or more, and the upper limit is preferably 50 µm or less, more preferably 20 µm or less, still more preferably 10 µm or less and even more preferably 5 µm or less, so that the particles do not feel frictional after application and drying on the skin, do not feel gritty upon application onto the skin, and are hardly removed upon rubbing on the skin, or the powder of the water-absorbing particles is not visible on the skin and does not seem white. The average particle diameter refers to the diameter of a particle not swollen with water, determined (in a cyclohexane solvent) with a light scattering particle-diameter measuring instrument (for example, LS-230 model manufactured by Coulter, Inc.).

The amount of water absorbed into the surface-hydrophobated water-absorbing polymer particles of this invention is preferably not lower than 5 g/g, more preferably not lower than 10 g/g. The upper limit is preferably not higher than 100 g/g, more preferably not higher than 50 g/g, still more preferably not higher than 30 g/g. In this range, a sufficient anti-perspiring ability can be achieved, and if the water-absorbing polymer absorbs sweat, it does not give slimy feel and is hardly released from the skin. The amount of water absorbed can be determined by adding 1000 ml water to 5 g sample, then suspending and stirring it for 30 minutes (100 rpm, 25°C), centrifuging it at 2,000 G for 30 minutes, discarding the supernatant gently, measuring the weight of the sample, and determining the amount of water absorbed, on the basis of the difference of this weight from the initial weight of the sample.

As is described later, the surface-hydrophobated water-absorbing polymer particles can be obtained by a method (A) of preparing surface-hydrophobated water-absorbing polymer particles by allowing a silicone compound having at least one kind of functional group, a fluorine-based surfactant etc., to be present during polymerization of a hydrophilic monomer, a method (B) of preparing surface-hydrophobated water-absorbing polymer particles from water-absorbing polymer particles after polymerization of a hydrophilic vinyl monomer or from previously obtained water-absorbing polymer particles such as a naturally occurring polymer, etc.

As the water-absorbing polymer used in the surface-hydrophobated water-absorbing polymer particles, a natural polymer, a semi-synthetic polymer or a synthetic polymer can be used insofar as it is a polymer having the action of absorbing water. To attain water-absorbing properties, it is preferably a polymer having a crosslinked structure, and such a polymer is a (co)polymer crosslinked by a crosslinking method described later or a (co) polymer having a crosslinkage via a hydrogen bond or hydrophobic bond, a crosslinkage derived from a partial crystalline structure or a crosslinkage derived from a helix structure etc. [(co)polymer means a polymer or copolymer.].

For example, the natural polymer and semi-synthetic polymer include starch, carrageenan, gelatin, agar, tragacanth gum, viscose, cellulose (for example, crystalline cellulose), methyl cellulose, ethyl cellulose, hydroxyethyl cellulose and carboxymethyl cellulose, or crosslinked products thereof, for example starch-(meth)acrylate graft copolymers (or its crosslinked products) [(meth)acrylate means acrylate, methacrylate or a mixture thereof.].

The synthetic polymer includes a crosslinked product of a (co)polymer of hydrophilic vinyl monomers such as anionic monomers or salts thereof, nonionic hydrophilic group-containing monomers, amino group-containing unsaturated monomers or neutralized products thereof or quaternarized products thereof. The hydrophilicity of the hydrophilic vinyl monomer means that the solubility in 100g of water (20°C) is preferably 6 wt.% or more, more preferably more than 20 wt.%. Examples of monomers used in production of the synthetic polymer include anionic monomers such as (meth) acrylic acid, maleic acid, fumaric acid, crotonic acid, itaconic acid, 2-(meth)acryloyl ethane sulfonic acid, 2-(meth)acryloyl propane sulfonic acid, 2-(meth)acrylamide-2-methyl propane sulfonic acid, vinyl sulfonic acid and styrene sulfonic acid or salts thereof; nonionic hydrophilic group-containing monomers such as (meth)acrylamide, N-substituted (meth)acrylamide, 2-hydroxyethyl (meth)acrylate, 2-hydroxypropyl (meth)acrylate, methoxypolyethylene glycol (meth)acrylate, polyethylene glycol(meth)acrylate, N-vinyl pyrrolidone and N-vinyl acetamide; and amino-group containing unsaturated monomers such as N,N-dimethylaminoethyl (meth)acrylate, N,N-dimethylaminopropyl (meth)acrylate and N,N-dimethylaminopropyl (meth)acrylamide or acid-neutralized product thereof, or quaternized products thereof. Preferable examples of the acid used in producing the acid-neutralized product include hydrochloric acid, sulfuric acid, nitric acid, acetic acid, formic acid, maleic acid, fumaric acid, citric acid, tartaric acid, adipic acid, lactic acid etc., and preferable examples of the quaternizing agent include alkyl halides such as methyl chloride, ethyl chloride, methyl bromide and methyl iodide, and standard alkylating agents such as dimethyl sulfate, diethyl sulfate and di-n-propyl sulfate. The counterion includes halogen ions such as chlorine and organic anions such as methosulfate. Unless the hydrophilicity of the resultant polymer is extremely inhibited, the hydrophobic vinyl monomers such as acrylates and styrene or derivatives thereof can also be simultaneously copolymerized in an amount of preferably 0 to 50% by weight, more preferably 0 to 20 wt.%, of the total monomers.

The hydrophobicity of the hydrophobic vinyl monomer means that the solubility in 100g of water (20°C) is preferably less than 6 wt.%. No lower limit is provided, but it may be 0.01 wt.% or more.

As the monomer components used, one or more of those enumerated above can be selected, but crosslinked (co)polymers of α,β-unsaturated carboxylic acid monomers such as (meth) acrylic acid, maleic acid, fumaric acid, crotonic acid, itaconic acid or salts thereof are preferable because of their high water-absorbing ability and easy availability. In addition to the α,β-unsaturated carboxylic acid monomers, other monomers can also be copolymerized.

To improve the water-absorbing ability, the amount of the hydrophilic vinyl monomers blended is preferably at least 50% by weight, more preferably at least 70% by weight and particularly preferably at least 90% by weight of the total monomers constituting the water-absorbing polymer particles.

The water-absorbing polymer particles are preferably a crosslinked polymer or copolymer of hydrophilic vinyl monomers and/or salt thereof, more preferably a crosslinked polymer or copolymer of α,β-unsaturated carboxylic acid monomers and/or salts thereof, particularly preferably crosslinked poly(meth)acrylate. These water-absorbing polymers can be used alone or in combination thereof.

The "salt" includes e.g. alkali metal salts (sodium salt, potassium salt, lithium salt etc.), alkaline earth metal salts (calcium salt, magnesium salt, barium salt etc.) and ammonium salts (quaternary ammonium salt, quaternary alkyl ammonium salt etc.). Among these, the sodium salt is most inexpensive and preferable. Here, the degree of neutralization of the water-absorbing polymer particles is preferably 0.01 to 100%, more preferably 1 to 99% and particularly preferably 40 to 95% based on the number of moles of the acid group in the water-absorbing polymer. In this invention, the "degree of neutralization" refers to the ratio (on a molar basis) of the salt-forming acid group to the acid group in the water-absorbing polymer, that is, (number of moles of the salt-forming acid group) / (number of moles of the salt-formable free acid plus the salt-forming acid group) × 100 (%).

The method of forming the crosslinked (co)polymers described above involves covalent crosslinking by (a) self-crosslinking during polymerization, (b) copolymerization with a multifunctional monomer, and (c) irradiation with radiation, or ionic crosslinking via (d) polyvalent metal ion. Among these, (b) is preferable from the viewpoint of easy production and stability of the crosslinked structure, and the multifunctional monomer includes both a crosslinking vinyl monomer having at least 2 reactive unsaturated groups in the molecule and a compound having at least 2 functional groups other than unsaturated groups in the molecule.

Among these crosslinking vinyl monomers having at least 2 reactive unsaturated.groups in the molecule, ethylene glycol di(meth)acrylate, polyethylene glycol di(meth)acrylate, divinyl benzene, pentaerythritol triallyl ether, pentaerythritol tetraallyl ether, and methylene bisacrylamide are preferable.

The compound having at least 2 functional groups other than unsaturated groups in the molecule is more preferably ethylene glycol diglycidyl ether or polyethylene glycol diglycidyl ether.

The amount of the crosslinking agent added is varied depending on the type of the crosslinking agent and the crosslinking method, but is preferably 0.001 part by weight or more, more preferably 0.01 part by weight or more, particularly preferably 0.1 part by weight or more, relative to 100 parts by weight of the total monomers constituting the water-absorbing polymer particles, and the upper limit is preferably 20 parts by weight or less, more preferably 10 parts by weight or less, particularly preferably 5 parts by weight or less. When the amount of the crosslinking agent is 0.001 part by weight or more, the amount of water absorbed can be maintained due to a low content of water-soluble components in the resulting water-absorbing polymer, while when its amount is 20 parts by weight or less, the density of crosslinkages is suitable and the amount of water absorbed into the resultant water-absorbing polymer is sufficient.

Hereinafter, preferable surface-hydrophobated water-absorbing polymer particles are specifically described.

### (1) Surface-hydrophobated water-absorbing polymer particles wherein the surfaces of water-absorbing polymer particles were coated with a silicone compound having at least one kind of functional group

The silicone compound having at least one kind of functional group is a silicone compound having at least 2 silicon atoms, having at least one kind of functional group capable of chemical bonding, preferably covalent bonding and/or ionic bonding, to the surfaces of water-absorbing polymer particles.

In the surface-hydrophobated water-absorbing polymer particles, the surfaces of the water-absorbing polymer particles have been coated preferably via chemical bonding with a silicone compound having at least one kind of functional group. The silicone compound can thereby be stably present on the surfaces of the water-absorbing polymer particles, even upon incorporation into cosmetics. The presence of such chemical bonding can be confirmed by the fact that the silicone compound is present on the water-absorbing polymer particles even if the silicon-modified polymer particles are subjected 3 times to the washing step of treating, with chloroform (10% by weight relative to chloroform) for 2 hours under stirring (30 rpm, 50°C) and then centrifuged.

The water-absorbing polymer particles are preferably coated thereon with the silicone compound to such an extent that the stickiness of the surfaces of the water-absorbing swollen particles can be suppressed.

Assuming that the amount of the total polymer particles (including the silicone compound) is 100 parts by weight, the lower limit of the amount of the silicone compound having at least one kind of functional group is preferably 0.1 part by weight or more, more preferably 0.5 part by weight or more, still more preferably 1 part by weight or more. The upper limit is preferably 30 parts by weight or less, more preferably 10 parts by weight or less, still more preferably 5 parts by weight or less.

To prevent gel blocking among polymer particles or sticky feeling during use, the silicone compound having at least one kind of functional group is preferably hydrophobic. In particular, when the silicone compound is the one having plural kinds of functional groups and having a functional group not contributing to the reaction, the silicone compound is preferably hydrophobic.

As used herein, the hydrophobic silicone compound having at least one kind of functional group refers to the one whose solubility in 100 g water at 25°C is 10% by weight or less, preferably 1% by weight or less, more preferably 0.5% by weight or less and particularly preferably 0.1% by weight or less. The lower limit of the solubility is not particularly present, but may be at least 0.01% by weight.

The weight average molecular weight of the silicone compound having at least one kind of functional group is preferably 1000 to 500,000, more preferably 3000 to 200,000 and particularly preferably 10,000 to 200,000. Measurement of this weight average molecular weight is conducted by gel permeation chromatography (GPC), using polystyrene as standard and chloroform as eluent.

The functional group is preferably at least one kind of functional group selected from the group consisting of an amino group, an ammonium group, a hydroxy group, a carboxy group, an epoxy group and a radical-polymerizable unsaturated group, more preferably an amino group and/or an ammonium group. These groups may be located at a side chain, one end and/or both ends of siloxane, or may be a mixture thereof.

Typical examples of the silicone compound having at least one kind of functional group used in this invention are shown below.

### (1-1) Silicone compound having an amino group and/or an ammonium group (referred to hereinafter as amino-modified silicone)

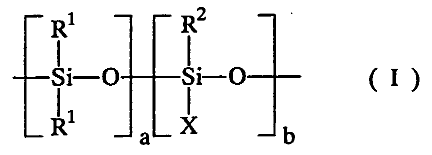

wherein R¹ represents a hydrogen atom or a C₁₋₆ hydrocarbon group, and a plurality of R¹s may be the same or different; R² represents R¹ or X whereupon X is a reactive functional group represented by -R³-Z (R³ represents a direct bond or a C₁₋₂₀ divalent hydrocarbon group, and Z represents a primary to tertiary amino group-containing group or a quaternary ammonium group-containing group); and a is a number of 2 or more, and b is a number of 1 or more.

In the general formula (I), R¹ groups independently represent a hydrogen atom or a C₁₋₆ hydrocarbon group, for example an alkyl group or phenyl group, preferably a methyl group or ethyl group, more preferably a methyl group. R³ is preferably a C₁₋₆ linear or branched alkylene group, and includes a methylene group, ethylene group, trimethylene group, propylene group, tetramethylene group etc., more preferably a trimethylene group or propylene group. a and b each represent the number of polymerizing repeating units. Preferably, a is a number of 2 to 1000, and b is a number of 1 to 50. Z is preferably an amino group- or ammonium group-containing group represented by the general formula (II) or (III) below. 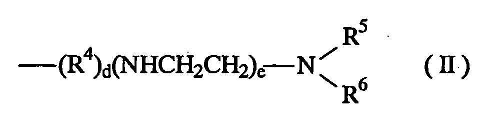 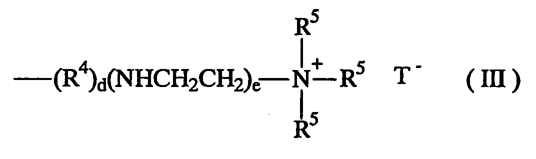 wherein R⁴ represents

―OCH₂CH₂― ,

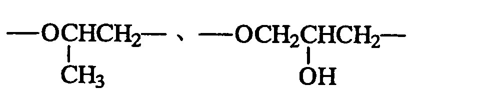

or 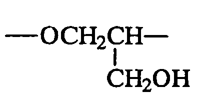 R⁵ and R⁶ each represent a hydrogen atom or a monovalent hydrocarbon group, d and e each represent an integer of 0 to 6, and T⁻ represents a halogen atom or an organic anion.

In the general formula (III), a plurality of R⁵s may be the same or different. Specific examples of T⁻ include halogen ions such as chlorine, iodine, bromine etc., and organic anions such as methosulfate, ethosulfate, methophosphate, ethophosphate etc.

In the general formula (I), the group X is preferably -(CH₂)₃-NH₂, -(CH₂)₃-N(CH₃)₂-, -(CH₂)₃-NH-(CH₂)₂-NH₂, -(CH₂)₃-NH-(CH₂)₂-N(CH₃)₂, or -(CH₂)₃-N⁺(CH₃)₃Cl⁻, more preferably -(CH₂)₃-NH-(CH₂)₂-NH₂.

The weight average molecular weight of the amino-modified silicone is preferably 3000 to 200, 000, and for easy reaction with anionic functional groups of the water-absorbing polymer and for hydrophobicity of the silicone compound, the amine equivalent thereof is preferably 250 to 10000 g/mol, more preferably 1000 to 5000 g/mol. That is, the amount of the amino group or ammonium group in the polymer is preferably 0.1 to 4 mmol/g, more preferably 0.2 to 1 mmol/g. The amine equivalent can be measured in a solvent such as ethanol by titration with hydrochloric acid of known concentration.

### (1-2) Silicon compound having a carboxy group (referred to hereinafter as carboxy-modified silicone)

The carboxy-modified silicone is preferably either a compound having a silicon atom and carboxy group bound via a saturated hydrocarbon, or an organosiloxane compound having a carboxyl group and/or a salt thereof bound to a silicon atom via a structure shown in the general formulae (IV) and/or (V), which is known in JP-A 2002-114849: 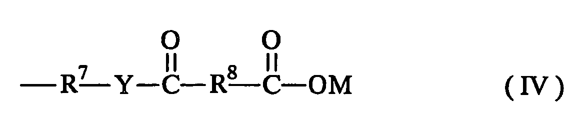 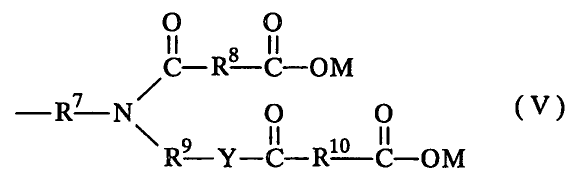 wherein R⁷, R⁸, R⁹ and R¹⁰ are the same or different and each represent a C₂₋₂₂ linear or branched alkylene or alkenylene group or an arylene group, which may have a substituent group containing a heteroatom; Y represents an -O- or -NH- group; and M represents a hydrogen atom, a metal, ammonium, C₁₋₂₂ alkyl or alkenyl ammonium, C₁₋₂₂ alkyl or alkenyl-substituted pyridinium, C₁₋₂₂ alkanol ammonium, or a basic amino acid.

Further, an amphoteric ionomer siloxane having two functional groups (carboxyl group and ammonium group) described in JP-A 6-1711 can also be preferably used.

The weight average molecular weight of the carboxy-modified silicone is preferably 3000 to 200,000. For easy reaction with cationic functional groups of the water-absorbing polymer and for hydrophobicity of the silicone compound, the carboxy equivalent thereof is preferably 250 to 10000 g/mol, more preferably 1000 to 5000 g/mol. That is, the amount of the carboxy group in the polymer is preferably 0.1 to 4 mmol/g, more preferably 0.2 to 1 mmol/g. The carboxy equivalent can be measured in a solvent such as ethanol by titration with sodium hydroxide of known concentration.

### (1-3) Silicone compound having a hydroxyl group (referred to hereinafter as hydroxyl-modified silicone)

The hydroxy-modified silicone includes the branched silicone of the general formula (VI), the both-terminal type silicone of the general formula (VII) and an alkylglyceryl ether-modified silicone of the general formula (VIII) disclosed in JP-A 5-112424. 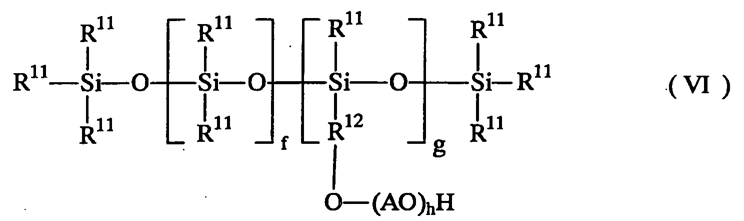 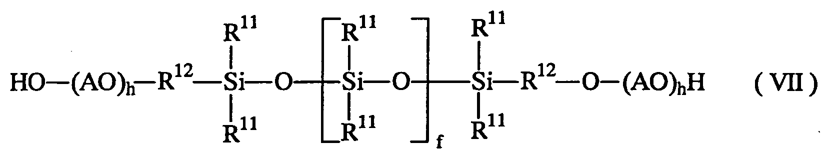 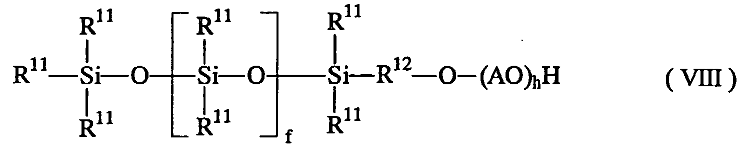 wherein R¹¹ groups may be the same or different and each represent a C₁₋₃ alkyl group, preferably a methyl group; R¹² groups may be the same or different and each represent a C₁₋₈ alkylene group, preferably a trimethylene group; AO groups may be the same or different and each represent an ethyleneoxy group or propyleneoxy group; f and g each are an integer of 1 or more; and h is an integer of 0 or 1 or more.

### (1-4) Silicone compound having an epoxy group (referred to hereinafter as epoxy-modified silicone)

The epoxy-modified silicone is preferably a silicone compound containing an epoxy group at one and/or both ends represented by the general formula (IX): 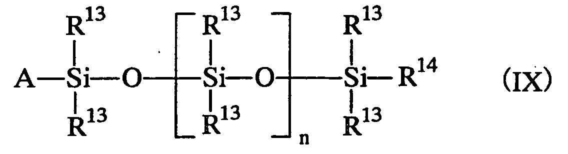 wherein R¹³ groups are the same or different and each represent a monovalent hydrocarbon group; R¹⁴ represents an epoxy-containing group or a monovalent hydrocarbon group; A represents an epoxy-containing group; and n is a number of 1 to 10000.

The monovalent hydrocarbon group represented by R¹³ includes an alkyl group such as methyl group and ethyl group, a cycloalkyl group such as cyclohexyl group, an aryl group such as phenyl group, and a fluorine atom-substituted alkyl group such as trifluoropropyl group. All R¹³ groups may be the same or different, but all R¹³ groups are desirably methyl groups.

In the general formula (IX), the epoxy-containing group represented by A is not particularly limited, but specifically includes the groups shown in the general formulae (X) to (XII): 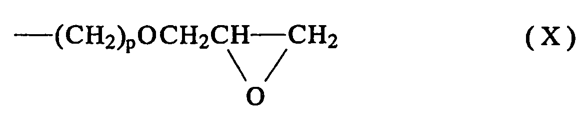 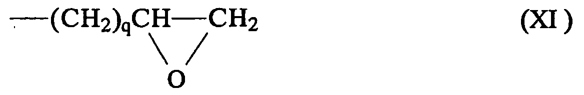 wherein p and q each are an integer of 1 or more. 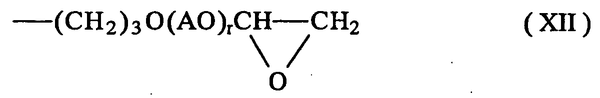 wherein r is an integer of 1 or more, and AO groups whose number is r are the same or different and each represent an ethyleneoxy group or propyleneoxy group.

In the general formula (IX), when R¹⁴ represents the same group as in R¹³, the compound is silicone containing an epoxy group at one end, while when R¹⁴ represents the same group as in A, the compound is silicone containing an epoxy group at both ends.

The epoxy-modified silicone is preferably silicone containing an epoxy group at one end, that is, silicone wherein R¹⁴ is the same group as in R¹³, wherein the hydrocarbon group is preferably an alkyl group such as methyl group and ethyl group, a cycloalkyl group such as cyclohexyl group, an aryl group such as phenyl group and a fluorine atom-substituted alkyl group such as trifluoropropyl group, more preferably a methyl group.

In the general formula (IX), n is preferably 1 to 500, more preferably 5 to 100 and particularly preferably 10 to 50. (1-5) Silicone compound having a radical polymerizable unsaturated group

Preferable examples of the silicone compound having a radical polymerizable unsaturated group include a polysiloxane compound having a radical polymerizable group at one end described in JP-A 11-181003.

### (2) Surface-hydrophobated water-absorbing polymer particles wherein the surfaces of water-absorbing polymer particles were coated with a polymer of a hydrophobic vinyl monomer

The surface-hydrophobated water-absorbing polymer particles are water-absorbing polymer particles wherein the surfaces of water-absorbing polymer particles were coated with a polymer of at least one kind of hydrophobic vinyl monomer.

At least a part of the surfaces of the water-absorbing polymer particles is coated to such an extent as to suppress the stickiness of the surfaces of the water-absorbing swollen particles.

The lower limit of the amount of the polymer of hydrophobic vinyl monomers in the surface-hydrophobated water-absorbing polymer particles is preferably 1 weight part or more, more preferably 5 weight parts or more, particularly preferably 10 weight parts or more, relative to 100 parts by weight of the water-absorbing polymer particles. The upper limit is preferably 1000 weight parts or less, more preferably 400 weight parts or less, particularly preferably 200 weight parts or less. This range is preferable because there is no sticky feeling, and the speed of water absorption is not particularly inhibited.

The hydrophobic vinyl monomer used in the present invention is a polymerizable monomer giving a hydrophobic polymer by polymerization, and can be polymerized by a polymerization method with a general radical polymerization initiator or irradiation with ultraviolet light.

The hydrophobic vinyl monomer is a monomer having the hydrophobicity defined above, and a usual radical polymerizable hydrophobic vinyl monomer is preferably used. Examples of the monomer include, for example, styrene, acrylates such as vinyl acetate, divinyl benzene, butyl acrylate, 2-ethylhexyl acrylate, cyclohexyl acrylate, decyl acrylate, lauryl acrylate, dodecenyl acrylate, myristyl acrylate, palmityl acrylate, hexadecenyl acrylate, stearyl acrylate, octadecenyl acrylate, behenyl acrylate etc., methacrylates such as butyl methacrylate, 2-ethylhexyl methacrylate, cyclohexyl methacrylate, decyl methacrylate, lauryl methacrylate, dodecenyl methacrylate, myristyl methacrylate, palmityl methacrylate, hexadecenyl methacrylate, stearyl methacrylate, octadecenyl methacrylate, behenyl methacrylate etc., fluorine-based monomers such as trifluoroethyl methacrylate, silicone macromonomers etc. These hydrophobic monomers can be used as a mixture of one or more thereof. Among these monomers, styrene and alkyl (meth)acrylates having a C₁₋₂₂ alkyl group are highly hydrophobic, easily available and preferable.

The weight average molecular weight of the hydrophobic polymer used in this invention is preferably 1000 to 500, 000, more preferably 3000 to 200,000 and particularly preferably 10,000 to 200,000. Measurement of this weight average molecular weight is conducted by gel permeation chromatography (GPC), using polystyrene as standard. The column used was Shodex KF-806 manufactured by Showa Denko K.K.

To improve the adhesion of the formed polymer to the water-adsorbing polymer, the hydrophilic vinyl monomer may be copolymerized to such an extent that the hydrophobicity of the resulting polymer is extremely inhibited.

Such hydrophilic vinyl monomer includes α,β-unsaturated carboxylic acids such as (meth)acrylic acid, maleic acid, itaconic acid etc., maleic anhydride, chloromethyl styrene, glycidyl (meth)acrylate, (meth)acryloyloxyethyl isocyanate, 3-(trimethoxysilyl)propyl (meth)acrylate, dimethylaminoethyl (meth)acrylate, hydroxyethyl (meth)acrylate, vinyl pyridine, (meth)acrylamide etc. These hydrophilic vinyl monomers can be used as a mixture of two or more thereof. The amount of the hydrophilic vinyl monomer used is preferably 0 to 50 weight%, more preferably 0 to 20 weight%, of the total monomers constituting the polymer of the hydrophobic vinyl monomer.

### (3) Surface-hydrophobated water-absorbing polymer particles wherein the surfaces of water-absorbing polymer particles were treated with a fluorine-based surfactant

A hydrophilic moiety of the fluorine-based surfactant can be any of four kinds of moieties, that is, anionic, nonionic, cationic and amphoteric, while a hydrophobic moiety of the surfactant can make use of a fluorocarbon chain or a perfluorocarbon chain. The fluorine-based surfactant preferably has a functional group capable of reacting with the surfaces of the water-absorbing particles, and the hydrophobic moiety is preferably anionic, cationic or amphoteric, more preferably cationic or amphoteric. The carbon chain in the hydrophobic moiety may be a linear or branched chain. For example, the following fluorine-based surfactants can be mentioned.

Fluoroalkyl (C₂ to C₁₀) carboxylic acids, disodium N-perfluorooctane sulfonyl glutamate, sodium 3- [fluoroalkyl (C₆ to C₁₁)oxy]-1-alkyl (C₃ to C₄) sulfonate, sodium 3-[ω-fluoroalkanoyl (C₆ to C₈)-N-ethylamino]-1-propane sulfonate, N-[3-(perfluorooctanesulfonamide) propyl]-N,N-dimethyl-N-carboxymethylene ammonium betaine, fluoroalkyl (C₁₁ to C₂₀) carboxylic acid, perfluoroalkyl carboxylic acid (C₇ to C₁₃), perfluorooctane sulfonic acid diethanol amide, perfluoroalkyl (C₄ to C₁₂) sulfonates (Li, K, Na), N-propyl-N- (2-hydroxyethyl) perfluorooctane sulfonamide, perfluoroalkyl (C₆ to C₁₀) sulfonamide propyl trimethyl ammonium salt, perfluoroalkyl (C₆ to C₁₀)-N-ethyl sulfonyl glycine salt (K), bis(N-perfluorooctylsulfonyl-N-ethylaminoethyl) phosphate, monoperfluoroalkyl (C₆ to C₁₆) ethyl phosphate, perfluoroalkyl quaternary ammonium iodide (trade name: Florade FC-135, a cationic fluorine-based surfactant manufactured by Sumitomo 3M Ltd.), perfluoroalkyl alkoxylate (trade name: Florade FC-171, a nonionic fluorine-based surfactant manufactured by Sumitomo 3M Ltd.) and potassium perfluoroalkyl sulfonates (trade names: Florade FC-95 and FC-98, anionic surfactants manufactured by Sumitomo 3M Ltd.).

### (4) Surface-hydrophobated water-absorbing polymer particles wherein the surfaces of water-absorbing polymer particles were treated with a silane coupling agent

As the silane coupling agent, a silane coupling described in JP-A 61-211305 can be used.

Preferable examples include γ-glycidoxy propyl trimethoxy silane, γ-glycidoxy propyl methyl diethoxy silane, γ-(2-aminoethyl)aminopropyl trimethoxy silane, γ-(2-aminoethyl) aminopropyl methyl diethoxy silane, γ-aminopropyl triemethoxy silane, γ-chloropropyl trimethoxy silane, γ-chloropropyl methyl dimethoxy silane etc.

### [Method of producing surface-hydrophobated water-absorbing polymer particles]

### (A) Method of preparing surface-hydrophobated water-absorbing polymer particles by allowing the silicone compound having at least one kind of functional group, a fluorine-based surfactant etc., to be present during polymerization of the hydrophilic vinyl monomer.

When a polymer of a hydrophilic vinyl monomer is used in the water-absorbing polymer particles, the hydrophilic vinyl monomer and crosslinking agent may be polymerized by any method, but a method of polymerizing an aqueous solution of the hydrophilic vinyl monomer (preferably at a concentration of 1 to 70% by weight) is preferable, and various methods such as aqueous solution polymerization, reverse phase suspension polymerization and pearl polymerization can be used. In particular, aqueous solution polymerization or reverse phase suspension polymerization is preferable in respect of operativeness for polymerization and the water-absorbing ability of the resultant water-absorbing polymer particles, and reverse phase suspension polymerization is particularly preferable in respect of a higher water-absorbing ability of the water-absorbing polymer particles. The temperature for polymerization of the hydrophilic vinyl monomer is preferably 20 to 120°C and the polymerization time is preferably 20 to 180 minutes.

As the polymerization initiator, a water-soluble radical polymerization initiator, for example a peroxide, hydroperoxide or an azo compound is known in a known amount. Two or more of the polymerization initiators can be mixed and used. Then they can be used as a redox type polymerization initiator by adding thereto chromium ion, sulfite, hydroxylamine, hydrazine or the like. If necessary, an oil-soluble radical polymerization initiator, for example a peroxide type initiator such as benzoyl peroxide, lauroyl peroxide, tert-butyl peroxy pivalate or diisopropyl peroxy dicarbonate, or an azo type initiator such as azobis(isobutyronitrile), azobis(2,4-dimethylvaleronitrile), azobis(dimethyl isobutyrate) or azobis(cyclohexane carbonitrile) can also be used.

The amount of the water-soluble polymerization initiator is preferably 0.03 to 5 wt.%, more preferably 0.1 to 2 wt.%, based on the amount of the hydrophilic vinyl monomer.

A dispersant is used to disperse and stabilize the hydrophilic vinyl monomer stably in the oil phase (solvent). The silicone compound having at least one kind of functional group or the fluorine-based surfactant is preferably used as the dispersant. It may be combined with another dispersant described later. Among the silicone compounds, the dispersant is preferably a silicone compound having at least one kind of functional group selected from the group consisting of an amino group, an ammonium group, a hydroxy group and a carboxy group.

The dispersant is present in an amount of preferably 0.5 to 30 weight%, more preferably 1 to 10 weight%, still more preferably 1 to 7 weight%, relative to 100 weight% of the total vinyl monomers constituting the water-absorbing polymer particles.

The other dispersant which can be used in combination includes a general anionic, cationic, nonionic or amphoteric surfactant or a natural, semi-synthetic or synthetic polymer. Examples thereof include an anionic surfactant such as sodium polyoxyethylene dodecyl ether sulfate and sodium dodecylether sulfate, a cationic or amphoteric surfactant such as trimethylstearylammonium chloride and carboxymethyldimethylcetyl ammonium, a sucrose fatty ester such as sucrose monostearate and sucrose dilaurate, a sorbitan ester such as sorbitan monostearate, a nonionic surfactant such as polyoxyalkylene adducts of sorbitan esters, such as polyoxyethylenesorbitan monostearate, a natural or semi-synthetic polymer such as cellulose derivatives such as starch or derivatives thereof, cellulose ethers such as ethyl cellulose and cellulose esters such as cellulose acetate, and a synthetic polymer such as polyvinyl alcohol or derivatives thereof, maleic group-containing polybutadiene and a quaternary salt of styrene-dimethylaminoethyl methacrylate.

The solvent used in reverse phase suspension polymerization is preferably a hydrocarbon type solvent or silicone type solvent or a mixture thereof. Examples of the hydrocarbon type solvent include aliphatic hydrocarbons such as hexane, heptane, dodecane, cyclohexane, methyl cyclohexane, isooctane and hydrogenated triisobutylene and aromatic hydrocarbons such as benzene, toluene, xylene and ethyl benzene, and examples of the silicone type solvent include octamethyl cyclotetrasiloxane, decamethyl cyclopentasiloxane, hexamethyl disiloxane and octamethyl trisiloxane. Among these solvents, hexane and cyclohexane are particularly preferable.

Before water or solvent in the reaction system is distilled away after polymerization, the surface of particle may be crosslinked by adding a crosslinking agent later and reacting it under heating preferably at 40 to 150°C.

### (B) Method of preparing surface-hydrophobated water-absorbing polymer particles from water-absorbing polymer particles after polymerization of a hydrophilic vinyl monomer or from previously obtained water-absorbing polymer particles such as a naturally occurring polymer

The method is preferably a production method which includes heating water-absorbing particles and a silicone compound having at least one kind of functional group in the presence of a fluorine-based surfactant or a silane coupling agent, in the presence of water preferably a solvent, to which a crosslinking agent and/or a radical polymerization initiator is added if necessary. By the presence of water, functional groups in the water-absorbing polymer particles are dissociated to react readily with functional groups in the silicone compound having at least one kind of functional group, the fluorine-based surfactant or the silane coupling agent. The amount of the silicone compound having at least one kind of functional group, the fluorine-based surfactant or the silane coupling agent is preferably 0.001 part by weight or more, more preferably 0.01 part by weight or more, particularly preferably 0.1 part by weight or more, relative to 100 parts by weight of the water-absorbing polymer (particles), and the upper limit is preferably 20 parts by weight or less, more preferably 10 parts by weight or less, particularly preferably 5 parts by weight or less.

As the solvent, the above-mentioned hydrocarbon type solvent or silicone type solvent or a mixture thereof is preferably used. The amount of water in the reaction system is preferably 1 to 200 parts by weight, more preferably 10 to 100 parts by weight, relative to 100 parts by weight of the water-absorbing polymer (particles). After the reaction, the water is removed, for example, by distillation. When the crosslinking agent is used, the crosslinking agent is allowed to be present in an amount of 0.01 to 10 parts by weight, more preferably 0.01 to 5 parts by weight, still more preferably 0.01 to 3 parts by weight, relative to 100 parts by weight of the water-absorbing polymer (particles).

When the silicone compound having a radically polymerizable unsaturated group is used, the above-mentioned oil-soluble radical polymerization initiator is preferably allowed to be coexistent. The silicone compound having at least one kind of functional group or the fluorine-based surfactant can be added directly or as a solution wherein the silicone compound or the fluorine-based surfactant has been solubilized or dispersed by emulsification in an organic solvent, a surfactant or a dispersant, or in the form of a spray depending on the case. Heating for facilitating surface treatment is preferably in the range of 40 to 150°C.

The silicone compound having at least one kind of functional group, the fluorine-based surfactant or the silane coupling agent may be mixed with the water-absorbing polymer before or after disruption of the polymer, but it is more preferable that the water-absorbing polymer is disrupted to form water-absorbing polymer particles whose water content is regulated in a suitable range by drying if necessary, and the water-absorbing polymer particles are mixed in e.g. a kneader with the silicone compound having at least one kind of functional group.

The water-absorbing polymer particles coated with the polymer of the hydrophobic vinyl monomer is preferably the one obtained by adding the hydrophobic vinyl monomer and the above-mentioned oil-soluble polymerization initiator to a reverse phase suspension polymerization solution during or after polymerization of the water-absorbing polymer particles, that is, slurry having the water-absorbing polymer particles dispersed in a solvent, followed by polymerizing the monomer. The solvent is preferably the above-mentioned hydrocarbon type solvent or silicone type solvent or a mixture thereof.

The amount of the water-soluble polymerization initiator is preferably 0.03 to 5% by weight, more preferably 0.1 to 2% by weight, based on the amount of the hydrophobic vinyl monomer. The polymerization initiator is previously mixed with other components and used as a solution, and for the purpose of reducing the remaining monomer, it may be diluted with a solvent etc., during polymerization and added all at once or continuously. The polymerization temperature is preferably 20 to 120°C, and the polymerization time is preferably 20 to 180 minutes.

The surface-hydrophobated water-absorbing polymer particles used in the present invention are preferably those obtained by polymerization of a hydrophilic vinyl monomer and a crosslinking agent in the presence of a silicone compound having at least one kind of functional group or a fluorine-based surfactant by a reverse phase suspension polymerization method, those obtained by heating water-absorbing polymer particles, a silicone compound having at least one kind of functional group, a fluorine-based surfactant or a silane coupling agent, in the presence of water, and those obtained by reverse phase suspension polymerization of a hydrophilic vinyl monomer and a crosslinking agent in the presence of a water-soluble polymerization initiator and a dispersant, and during or after polymerization, adding a hydrophobic vinyl monomer and an oil-soluble polymerization initiator, followed by polymerization thereof.

### [Emulsified cosmetic composition]

The emulsified cosmetic composition of the present invention is preferably an antiperspirant and can be used as a skin lotion, cream, milky location or the like. Particularly, it is preferably used as a body lotion. The emulsified cosmetic composition may be an oil-in-water type (O/W) or water-in-oil type (W/O), but is preferably the O/W type because it spreads well, gives refreshing feel and improves anti-perspiring performance. The emulsified cosmetic composition is preferably the O/W type particularly for recovery of water absorptivity of the surface-hydrophobated water-absorbing polymer particles by evaporation of water from the surface-hydrophobated water-absorbing polymer particles in the emulsified cosmetic composition of the present invention.

The emulsified cosmetic composition of the present invention contains the surface-hydrophobated water-absorbing polymer particles in an amount of preferably 0.2 to 30% by weight, more preferably 0.5 to 10% by weight, particularly preferably 1 to 5% by weight (content in a state not swollen with water).

In the emulsified cosmetic composition of the present invention, the surface-hydrophobated water-absorbing polymer particles are preferably swollen with water in the composition, and the degree of swelling is preferably 5 to 100 g/g, more preferably 7 to 75 g/g, still more preferably 10 to 50 g/g. The particle diameter is preferably 0.8 to 100 µm, more preferably 1.5 to 50 µm, still more preferably 3 to 20 µm.

For attaining suitable viscosity, the emulsified cosmetic composition of the present invention contains water in an amount of 30% by weight or more, more preferably 50% by weight or more, still more preferably 60% by weight or more, or preferably 95% by weight or less, more preferably 90% by weight or less, still more preferably 85% by weight or less.

Because the composition of the present invention is an emulsified cosmetic composition, the composition preferably contains an emulsifying agent and an oil as described below.

The emulsifying agent used in the present invention includes aqueous substances having an oil-holding ability, such as an anionic surfactant, a cationic surfactant, an amphoteric surfactant, a nonionic surfactant, and an alkyl-added carboxy vinyl polymer, and the emulsifying for silicone oil includes a silicone-based surfactant, and one or more of these surfactants can be used.

Specific examples of the anionic surfactant include salts of aliphatic acids such as stearic acid, lauric acid, myristic acid, behenic acid, isostearic acid, oleic acid, resin acid, 12-hydroxystearic acid etc., salts of acylglutamic acid, salts of alkyl phosphoric acids, salts of polyoxyalkyl ether sulfuric acids, etc. , and the salts include alkali metal (sodium and potassium) salts, triethanol amine salts etc.

Specific examples of the cationic surfactant include alkyl amine salts, alkyl quaternary ammonium salts etc. Specific examples of the amphoteric surfactant include lecithin, phospholipids etc.

Examples of the nonionic surfactant include polyoxyethylene castor oil, polyoxyethylene hardened castor oil, and derivatives thereof; polyoxyethylene sorbitan fatty esters such as polyoxyethylene sorbitan monostearate, polyoxyethylene sorbitan tetraoleate etc.; polyoxyethylene glyceryl fatty esters such as polyoxyethylene glyceryl monoisostearate, polyoxyethylene glyceryl triisostearate etc.; polyoxyethylene-added surfactants such as polyoxyethylene alkyl or aryl ethers such as polyoxyethylene hexyldecyl ether, polyoxyethylene octyldodecyl ether, polyoxyethylene lauryl ether, polyoxyethylene cetyl ether, polyoxyethylene stearyl ether, polyoxyethylene nonylphenyl ether etc., as well as polyglycerin alkyl ether, polyglycerin fatty ester, sucrose fatty ester etc.

The emulsifying agent for silicone oil includes polyether-modified silicone, polyether/alkyl-modified silicone, glyceryl ether-modified silicone etc.

The emulsified cosmetic composition of the present invention is blended preferably with the nonionic surfactant in respect of safety and feel in use. This surfactant is contained preferably in an amount of 0.1 to 15% by weight, preferably 0.2 to 5% by weight, more preferably 0.3 to 2% by weight.

The emulsified cosmetic composition of the present invention preferably contains oils. Hydrocarbon-based oils include, for example, hydrocarbons such as liquid paraffin, paraffin wax, ceresin, squwalane etc.; wax such as beeswax, spermaceti, carnauba wax etc.; naturally occurring animal and vegetable fats and oils such as olive oil, camellia oil, jojoba oil, lanoline etc. ; ester oils such as octyldodecyl myristate, neopentyl glycol dioctanoate etc.; higher alcohols such as capryl alcohol, lauryl alcohol, myristyl alcohol, cetyl alcohol, stearyl alcohol, cholesterol etc.; aliphatic acids; triglycerides such as diglyceride, trioctanoic glyceride, etc. These hydrocarbon-based oils are incorporated in an amount of preferably 0.5 to 50% by weight, more preferably 1 to 30% by weight, still more preferably 2 to 15% by weight, into the cosmetic composition.

The emulsified cosmetic composition of the present invention preferably contains silicone oil in addition to the above-mentioned hydrocarbon-based oil. The silicone oil includes cyclic silicone such as octamethyl cyclotetrasiloxane, decamethyl cyclopentasiloxane, dodecamethyl cyclohexasiloxane etc., linear silicone such as dimethyl polysiloxane, methylphenyl polysiloxane etc., and solid or liquid silicone oil such as amino-modified silicone, polyether-modified silicone, methylphenyl polysiloxane, aliphatic acid-modified silicone, alcohol-modified silicone, alkoxy-modified silicone, fluorine-modified silicone, alkyl-modified silicone etc. Preferable among these are linear silicone and cyclic silicone, and those having a viscosity (25°C) of not higher than 1000 mm²/s can be preferably used without greasy feeling. These silicone oils are incorporated in an amount of preferably 0.2 to 30% by weight, more preferably 0.4 to 15% by weight, still more preferably 0.8 to 8% by weight, into the cosmetic composition.

When the cosmetic composition of the present invention contains a water-soluble polymer if necessary, the emulsion stability is further preferably improved. The water-soluble polymer used herein includes, for example, naturally occurring water-soluble polymers such as guar gum, quince seed, carrageenan, locust bean gum, gum arabic, tragacanth, pectin, mannan, starch, sodium alginate, sodium hyaluronate, xanthane gum, pullulan dextran, curd run, collagen, keratin, casein, albumin, gelatin, chondroitin sulfuric acid, chitin, agar, gelatin etc.; semi-synthetic water-soluble polymers such as cationized cellulose, hydroxyethyl cellulose, hydroxypropyl cellulose, hydroxypropylmethyl cellulose, hydroxyethyl cellulose, hydroxypropyltrimethyl ammonium chloride ether, carboxymethyl cellulose, dextran sulfuric acid, carboxymethyl chitin, soluble starch, carboxymethyl starch etc.; and synthetic water-soluble polymers such as carboxyvinyl polymer (polyacrylate), alkyl-added carboxyvinyl polymer, alginic propylene glycol, polyvinyl alcohol, polyvinyl pyrrolidone, polyvinyl methyl ether, polyethylene glycol etc. The water-soluble polymer is particularly preferably a polyacrylate (sodium salt, potassium salt).

The water-soluble polymer is preferably a polymer having a solubility of 1% by weight or more in water at 25°C. This solubility can be confirmed when at least one of light transmissions, at wavelengths of 500 and 800 nm respectively, of 1 weight% aqueous solution of the water-soluble polymer measured at 25°C in a quartz cell having a light path length of 10 mm is 60% or more. The water-soluble polymer may be a partially crosslinked polymer.

The content of the water-soluble polymer in the cosmetic composition of the present invention is preferably 0.01 to 5.0% by weight, more preferably 0.02 to 3.0% by weight.

The cosmetic composition of the present invention preferably contains a humectant. The humectant includes polyhydric alcohols such as glycerin, 1,3-butylene glycol, propylene glycol, polyethylene glycol, sorbitol etc., and sodium lactate, sodium 2-pyrrolidone-5-carboxylate, sodium hyaluronate etc. Among these compounds, glycerin is particularly preferable. The amount of the humectant incorporated into the cosmetic composition of the present invention is preferably 0.5 to 30% by weight, more preferably 1 to 20% by weight.

Depending on the form, type etc. of the cosmetic composition of the present invention, other generally used components can be further incorporated into the cosmetic composition in such a range that the effect of this invention is not hindered.

Such cosmetic components include e.g. extender pigments such as mica, talc, sericite, kaolin, polymethylsilyl sesquioxane and barium sulfate; inorganic pigments such as titanium oxide, zinc white and iron oxide; organic powders such as nylon powders; powders whose surface was rendered hydrophobic by treating these powders with silicone, metal soap or N-acyl glutamic acid; efficacious components such as whitening agent, analgesic antiinflammatory agents, anti-itching agents, astringents, skin softening agents and hormones; a thickener such as water-swelling clay minerals such as bentonite, hectorite and smectite having an ability to exchange a cation; ethanol; and other components such as emulsion stabilizer, sterilizers, chelating agents, UV protecting agents, pH adjusting agents, preservatives, coloring matters and perfumes.

When the viscosity of the emulsified cosmetic composition of the present invention is too low, the composition upon application onto the skin drops easily from the skin, while when the viscosity is too high, the composition hardly spreads upon application onto the skin, and therefore the viscosity is preferably 1,000 to 500,000 mPa·s, more preferably 3,000 to 200, 000 mPa·s, still more preferably 8,000 to 80,000 mPa·s. The viscosity can be determined by the following method.

### <Method of measuring viscosity>

A cosmetic composition regulated at 25°C is measured at a number of revolutions of 10 rpm with jig D by a t-type viscometer (model RVT, manufactured by Brookfield).

The pH value of the emulsified cosmetic composition of the present invention is preferably 4 to 9, more preferably 5 to 8, near to the weakly acidic pH of the surface of the human skin.

The cosmetic composition of this invention is produced according to a conventional method.

The emulsified cosmetic composition of the present invention contains the surface-hydrophobated water-absorbing polymer particles swollen with water so that after application onto the skin, water is evaporated from the surface-hydrophobated water-absorbing polymer particles in the emulsified cosmetics, and by this evaporation, the surface-hydrophobated water-absorbing polymer particles are considered to restore water absorptivity to absorb sweat. The surface-hydrophobated polymer particles are not sticky even after absorption of sweat and are preferable in feel. Accordingly, the emulsified cosmetics of the present invention, while having moisten feel, can prevent stickiness due to perspiration, and have preferable feel in use.

### Examples

In the following examples, "%" refers to "% by weight" unless otherwise specified.

### Synthesis Example 1

300 g methacrylic acid (Mitsubishi Rayon Co., Ltd.) and 135 g ion-exchange water were placed in a 2-L beaker and then neutralized to a degree of 75 % by adding 348 g of 30% aqueous sodium hydroxide solution dropwise thereto under cooling with stirring, and then a solution of 1.2 g potassium persulfate (0.4% relative to methacrylic acid) dissolved in 24.3 g ion-exchange water and a crosslinking agent ethylene glycol diglycidyl ether (trade name, Denacol EX810, Nagase Kasei Co. , Ltd.), 15.0 g (5.0% relative to methacrylic acid), were added thereto, and the mixture was uniformly dissolved. The resultant solution was added to a solution obtained by dissolving 15 g amino-modified silicone (XF42-703 produced by GE Toshiba Silicones Co., Ltd.; viscosity (25°C), 1000 mm²/s; amine equivalent, 1500 g/mol) in 1500 ml cyclohexane in a 2-L beaker, and the mixture was stirred vigorously for 5 minutes at a number of revolutions of 10000 rpm in a homomixer to produce a fine water-in-oil droplet dispersion. Then, 900 ml cyclohexane was introduced into a 5-L stainless steel reaction vessel equipped with a reflux condenser, then stirred at 350 rpm, and heated at 75°C in the system, and the atmosphere was replaced by nitrogen, followed by dropwise addition of water-in-oil droplet dispersion of the partially neutralized methacrylic acid to initiate polymerization. The whole of the droplet dispersion was added dropwise over 1.5 hours, and the reaction solution was aged for additional 4 hours at the reflux temperature. Thereafter, a dehydration tube was attached, the temperature was raised to remove 340 ml water, further 1300 ml cyclohexane was distilled away, and the reaction solution was left and cooled to complete synthesis. By evaporation into dryness under reduced pressure, 376 g dry white polymer in the form of fine powder was obtained. The average particle diameter of the resultant surface-hydrophobated water-absorbing polymer particles was 2.9 µm, and the amount of water absorbed was 15 g/g.

### Synthesis Example 2

A water-absorbing polymer was obtained in the same manner as in Synthesis Example 1 except that 15 g sugar esters (a mixture of Ryoto Sugar Esters S570 and S770 (trade name) in equal amounts, Mitsubishi Shokuhin Co., Ltd.) (5.0 % by weight relative to methacrylic acid) as a dispersion stabilizer were used in place of 15 g of the amino-modified silicone in Synthesis Example 1. 327 g of the resultant water-absorbing polymer was added to a solution obtained by dissolving 15 g amino-modified silicone (KF-864 produced by Shin-Etsu Chemical Co., Ltd.; viscosity (25°C), 1700 mm²/s; amine equivalent, 3800 g/mol) in 2000 ml cyclohexane, and the mixture was stirred at 350 rpm for 30 minutes under heating at 75°C. Then, 150 g ion-exchange water was added dropwise thereto, and after the mixture was stirred at 75°C for 30 minutes, a solution of a crosslinking agent ethylene glycol diglycidyl ether (trade name, Denacol EX810, Nagase Kasei Co. , Ltd.), 3.0 g (1.0% relative to methacrylic acid) in 20 g ion-exchange water was added dropwise thereto over 5 minutes. The reaction solution was aged for additional 4 hours at the reflux temperature. Thereafter, a dehydration tube was attached, the temperature was raised to remove 100 ml water, and the reaction solution was left and cooled to complete synthesis. By evaporation into dryness under reduced pressure, 375 g dry white polymer in the form of fine powder was obtained. The average particle diameter of the resultant surface-hydrophobated water-absorbing polymer particles was 3.0 µm, and the amount of water absorbed was 13 g/g.

### Synthesis Example 3

300 g methacrylic acid (Mitsubishi Rayon Co., Ltd.) and 135 g ion-exchange water were placed in a 2-L beaker and neutralized to a degree of 75% by adding 348 g of 30% aqueous sodium hydroxide solution dropwise thereto under cooling with stirring, and then a solution of 1.2 g potassium persulfate (0.4% relative to methacrylic acid) dissolved in 24.3 g ion-exchange water and 15.0 g of a crosslinking agent ethylene glycol diglycidyl ether (trade name, Denacol EX810, Nagase Kasei Co. , Ltd.) (5.0% relative to methacrylic acid) were added thereto, and the mixture was uniformly dissolved. The resultant solution was added to a solution obtained by dissolving 15 g sugar esters (a mixture of Ryoto Sugar Esters S570 and S770 (trade name) in equal amounts, Mitsubishi Shokuhin Co. , Ltd.) (5.0% relative to methacrylic acid) in 1500 ml cyclohexane in a 2-L beaker, and the mixture was stirred vigorously for 5 minutes at a number of revolutions of 10000 rpm in a homomixer to produce a fine water-in-oil droplet dispersion. Then, 900 ml cyclohexane was introduced into a 5-L stainless steel reaction vessel equipped with a reflux condenser, and stirred at 350 rpm and heated at 75°C in the system, and the atmosphere was replaced by nitrogen, followed by dropwise addition of the partially neutralized water-in-oil droplet dispersion of methacrylic acid to initiate polymerization. The whole of the droplet dispersion was added dropwise over 1.5 hours, and the reaction solution was aged for additional 4 hours at the reflux temperature. Thereafter, a dehydration tube was attached, the temperature was raised to remove 340 ml water, further 1300 ml cyclohexane was distilled away, and the reaction solution was left and cooled to complete synthesis. By evaporation into dryness under reduced pressure, 359 g dry white polymer in the form of fine powder was obtained. The average particle diameter of the resultant fine polymer particles was 2.9 µm, and the amount of water absorbed was 11 g/g.

### Examples 1 to 2 and Comparative Example 1

The water-absorbing polymer particles obtained in Synthesis Example 1 were used to prepare emulsified cosmetics having the compositions shown in Table 1 by the following method. The resulting emulsified cosmetics were evaluated for their performance by the following method. The results are shown in Table 1.

### <Method of preparing emulsified cosmetics>

Water, glycerin, methyl paraben, an acrylic acid polymer and Ceteareth-20 were introduced successively under stirring into a 200 mL container (referred to hereinafter as water tank) and then heated to 75°C. After 50% aqueous sodium hydroxide was added thereto, a dispersion of the water-absorbing polymer particles obtained in Synthesis Example 1 in decamethyl cyclosiloxane as dispersing medium was added thereto. Cetearyl alcohol, glyceryl dilaurate, myristyl stearate, cetyl alcohol and propyl paraben were placed in a 100-mL container and dissolved by heating at 75°C. Dimethicone was added to this container and mixed by a mixer, and the resultant mixture was added to the water tank. Ethanol, silicone powder, a preservative and a perfume were introduced into a 50-mL container, mixed under stirring and added to the water tank cooled to 45°C, and the mixture was cooled to room temperature to give emulsified cosmetics.

### <Method of evaluating performance>

### (1) Feeling upon application

0.5 g sample was applied onto the forearm, then evaluated by sensory test for the extendability and scored according to the following criteria.
○: Extends easily and spreads very well on the whole of the forearm.
×: Hardly extends and poor in spreading on the whole of the forearm.
××: Upon application, the sample flows and drops from the skin and cannot be spread.

### (2) Absence of sticky feel (when dried)

0.5 g sample was applied onto the forearm, left at room temperature for 10 minutes and dried. After drying, feeling on the skin was evaluated by sensory test and evaluated according to the following criteria:
○: Does not feel sticky and feels dry.
Δ: Feels slightly sticky.
×: Feels very sticky.

### (3) Absence of sticky feel (when perspired)

The absence of sticky feel of the sample when dried was evaluated as described above, and after perspired in a room with 75% RH at 40°C for 15 minutes, feel on the skin was evaluated by sensory test according to the following criteria:
○: Does not feel sticky and feels dry.
Δ: Feels slightly sticky.
×: Feels very sticky.

### (4) Absence of remaining whiteness after drying

After the above evaluation of sticky feel when perspiring, whether whiteness remained on the skin was evaluated.
○: There is no remaining whiteness.
Δ: Whiteness remains slightly.
×: Whiteness remains.

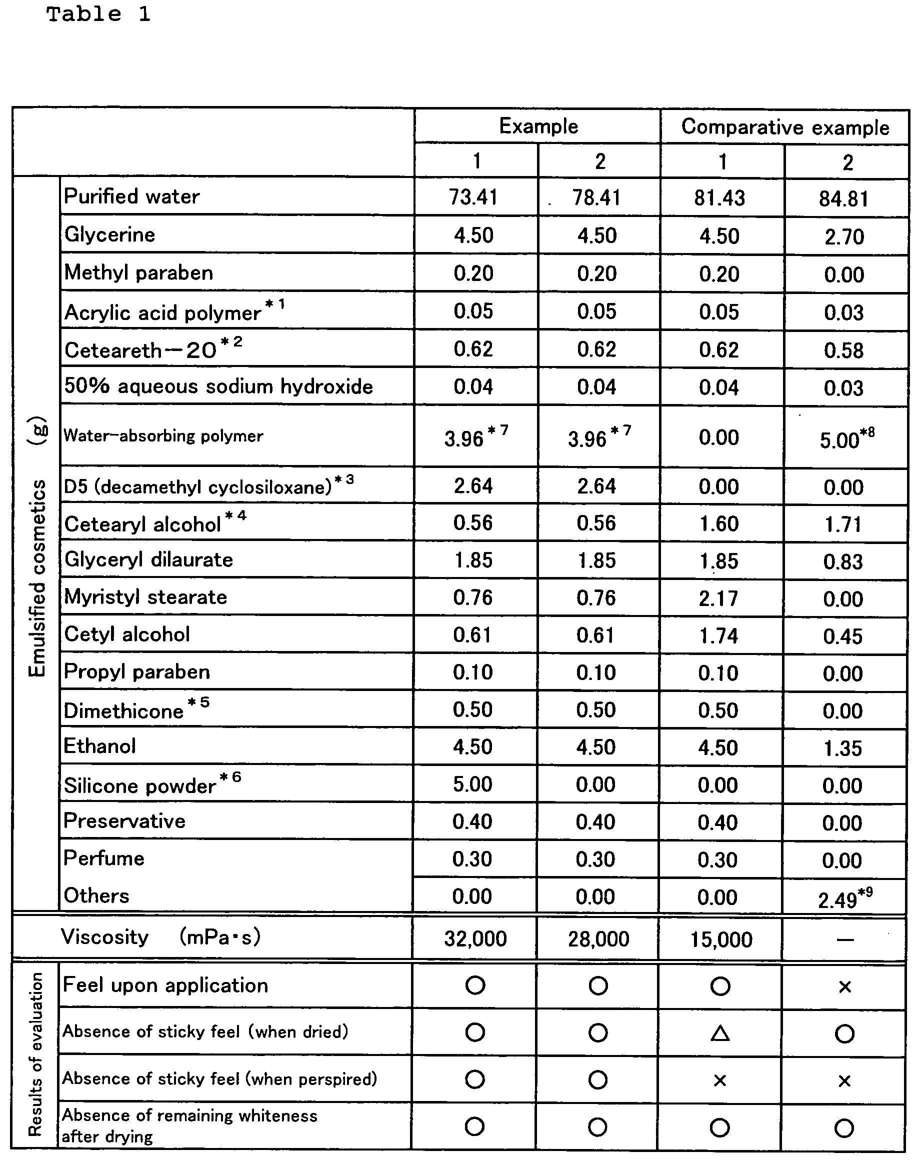

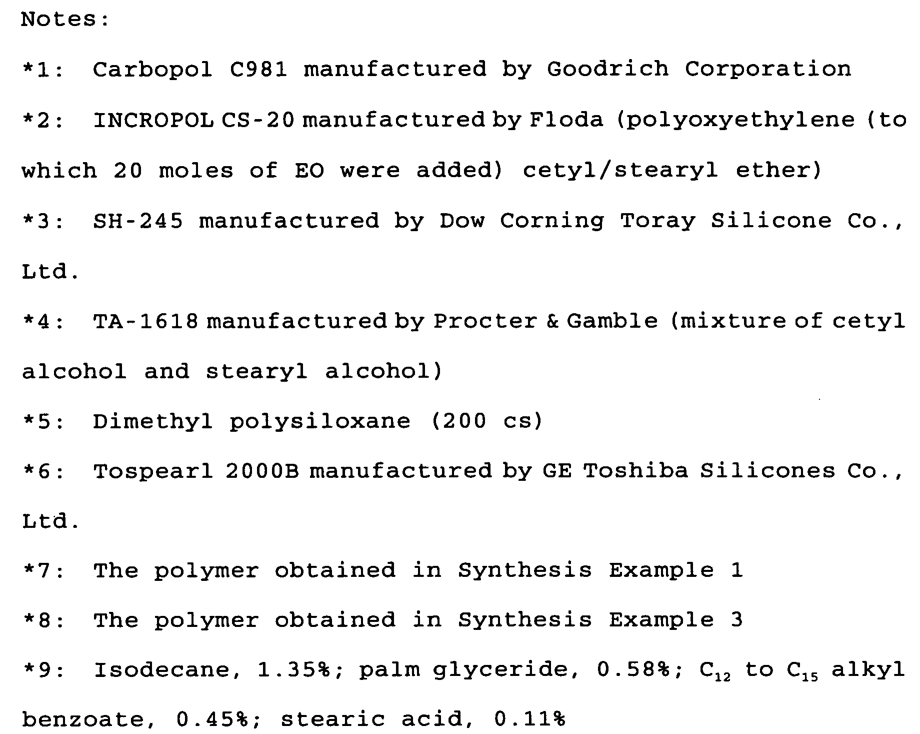

Hereinafter, formulation examples are shown.

### Formulation Example 1: Emulsified Cosmetics

| <Composition> | |
|---|---|
| Purified water | 64.58% |
| Glycerin | 4.50 |
| Methyl paraben | 0.20 |
| Acrylic acid polymer*¹ | 0.05 |
| Ceteareth-20*² | 0.62 |
| 50% aqueous sodium hydroxide | 0.04 |
| Dispersion of the water-absorbing polymer in Synthesis Example 1 | 16.50 |
| Cetearyl alcohol*⁴ | 0.28 |
| Glyceryl dilaurate | 1.85 |
| Myristyl stearate | 0.38 |
| Cetyl alcohol | 0.20 |
| Propyl paraben | 0.10 |
| Dimethicone*⁵ | 0.50 |
| Ethanol | 4.50 |
| Silicone powder*⁶ | 5.00 |
| Preservative | 0.40 |
| Perfume | 0.30 |

| | |
|---|---|
| Notes: *1, *2, *4, *5 and *6 are the same as in Table 1. *3: Dispersion of the water-absorbing polymer particles (D5 = 60 : 40 (ratio by weight)) in Synthesis Example 1 | |

### Formulation Example 2: Emulsified Cosmetics

| <Composition> | |
|---|---|
| Purified water | 73.41% |
| Glycerin | 4.50 |
| Methyl paraben | 0.20 |
| Acrylic acid polymer*¹ | 0.05 |
| Ceteareth-20*² | 0.62 |
| 50% aqueous sodium hydroxide | 0.04 |
| Dispersion of the water-absorbing polymer in Synthesis Example 2 | 6.60 |
| Cetearyl alcohol*⁴ | 0.56 |
| Glyceryl dilaurate | 1.85 |
| Myristyl stearate | 0.76 |
| Cetyl alcohol | 0.61 |
| Propyl paraben | 0.10 |
| Dimethicone*⁵ | 0.50 |
| Ethanol | 4.50 |
| Silicone powder*⁶ | 5.00 |
| Preservative | 0.40 |
| Perfume | 0.30 |

| | |
|---|---|
| Notes: *1, *2 and *4 to *6 are the same as in Table 1. *7: Dispersion of the water-absorbing polymer particles (D5 = 60 : 40 (ratio by weight))in Synthesis Example 2 | |

## Claims

1. An emulsified cosmetic composition comprising surface-hydrophobated water-absorbing polymer particles and water.

2. The emulsified cosmetic composition according to claim 1, wherein the average particle diameter of the surface-hydrophobated water-absorbing polymer particles is 0.1 to 50 µm.

3. The emulsified cosmetic composition according to claim 1 or 2, wherein the amount of water absorbed into the surface-hydrophobated water-absorbing polymer particles is 5 to 100 g/g.

4. The emulsified cosmetic composition according to any one of claim 1 to 3, which has a viscosity of 1,000 to 500, 000 mPa·s.

5. The emulsified cosmetic composition according to any one of claims 1 to 4, wherein the content of water is 30 to 95% by weight.

6. The emulsified cosmetic composition according to any one of claims 1 to 5, wherein the surface-hydrophobated water-absorbing polymer particles comprise water-absorbing polymer particles coated thereon with a silicone compound having at least one kind of a functional group.

7. The emulsified cosmetic composition according to any one of claims 1 to 6, which is used as an antiperspirant.

8. Use of an emulsified cosmetic composition comprising surface-hydrophobated water-absorbing polymer particles and water as cosmetics.

9. A method of antiperspiration, which comprises applying an emulsified composition comprising surface-hydrophobated water-absorbing polymer particles and water onto the skin.
